(19) Europäisches Patentamt
European Patent Office
Office européen des brevets

(11) EP 3 943 298 A1

(12) EUROPEAN PATENT APPLICATION
published in accordance with Art. 153(4) EPC

(43) Date of publication:
**26.01.2022 Bulletin 2022/04**

(21) Application number: **20773139.9**

(22) Date of filing: **12.03.2020**

(51) International Patent Classification (IPC):
***B32B 27/30*** *(2006.01)* ***B32B 27/00*** *(2006.01)*
***D06M 11/74*** *(2006.01)* ***D06M 15/263*** *(2006.01)*
***D06M 15/643*** *(2006.01)*

(52) Cooperative Patent Classification (CPC):
**B32B 27/00; B32B 27/30; D06M 11/74; D06M 15/263; D06M 15/643**

(86) International application number:
**PCT/JP2020/010726**

(87) International publication number:
**WO 2020/189479 (24.09.2020 Gazette 2020/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.03.2019 JP 2019050790**
**19.03.2019 JP 2019050791**

(71) Applicant: **Toray Industries, Inc.**
**Tokyo, 103-8666 (JP)**

(72) Inventors:
- **KATO, Tomohiro**
  **Otsu-shi, Shiga 520-8558 (JP)**
- **TAMAKI, Eiichiro**
  **Otsu-shi, Shiga 520-8558 (JP)**

(74) Representative: **Kador & Partner PartG mbB**
**Corneliusstraße 15**
**80469 München (DE)**

# (54) CONDUCTIVE SHEET

(57) A conductive sheet comprising a conductive substrate layer and a hydrogel layer formed of a silicone hydrogel having a polymer comprising a repeat unit (A) derived from a monomer represented by Formula (I) as a gel skeleton. Provided is a conductive sheet which can maintain adhesiveness and flexibility even when used for a long period of time and can attain high biocompatibility.

[Chemical Formula 1]

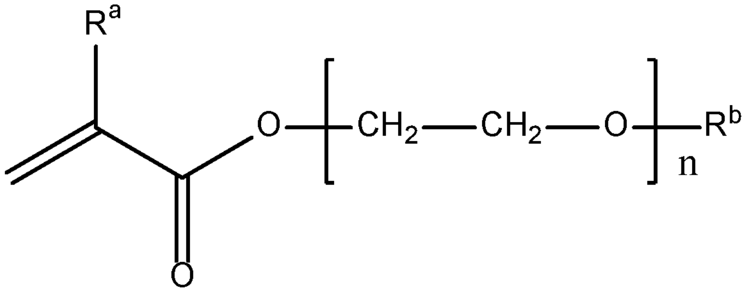

EP 3 943 298 A1

## Description

TECHNICAL FIELD

The present invention relates to a conductive sheet for use as an electrode and a method for producing the same.

BACKGROUND ART

A technique is known in which a conductive sheet is bonded as an electrode to an object to be measured and then information is electrically acquired. For example, for the purpose of medical care and health promotion, an electrode made of a conductive sheet is used as a contact point with a living body in a device for electrically acquiring an electrocardiogram, an electromyogram, an electroencephalogram, etc. According to the same principle, also when an electrochemical response of a material such as concrete, wood, a metal material, or a resin is measured, a conductive sheet is used as a contact point between a measurement device and an object to be measured. Also in an exercise instrument that contracts a muscle by electrical stimulation, a conductive sheet is used as a contact point with a living body. Furthermore, a conductive sheet is also used as a member for removing static electricity from a living body, preventing electrification of a precision instrument, and the like.

Such a conductive sheet may be provided with an adhesive material for securing adhesiveness to an object to be applied. For example, Patent Document 1 describes a sheet-shaped electrode for living bodies in which a hydrogel is disposed on an electrode or an electrode is embedded in a hydrogel. In addition, as a sheet electrode, for example, Patent Document 2 describes a method of manufacturing a conductive fiber by which method reduced graphene oxide with suppressed re-stacking can be fixed to a fiber, and a method of manufacturing a sheet electrode by which method reduced graphene oxide with suppressed re-stacking can be fixed to a fiber group molded in a sheet shape.

It is desired that the conductive sheet to be used for such applications can be used while being stably adhered to the skin for a long period of time without causing stuffiness or rash.

PRIOR ART DOCUMENTS

PATENT DOCUMENTS

Patent Document 1: WO 2014/157550
Patent Document 2: Japanese Patent Laid-open Publication No. 2015-221947

SUMMARY OF THE INVENTION

PROBLEMS TO BE SOLVED BY THE INVENTION

Patent Document 1 discloses that in the sheet-shaped electrode, it is intended to secure flexibility by using a hydrogel having a high water content and the water content of the hydrogel is preferably 60% by weight or more. However, such a hydrogel having a high water content has a problem that when used for a long period of time, the hydrogel dries to greatly shrink and stiffen and its adhesiveness and flexibility are easily impaired. The sheet-shaped electrode of Patent Document 2 has a problem that it cannot be used in adhesion to, for example, the surface of a living body because it has no adhesiveness to the skin.

An object of the present invention is to provide a conductive sheet which can maintain adhesiveness and flexibility even when used for a long period of time and can also exhibit high biocompatibility.

SOLUTIONS TO THE PROBLEMS

In order to solve the above problems, the present inventors have made intensive efforts and found that a conductive sheet having the following configuration is useful.

The present invention is a conductive sheet comprising a conductive substrate layer and a hydrogel layer formed of a silicone hydrogel having a polymer comprising a repeat unit (A) derived from a monomer represented by Formula (I), hereinafter described, as a gel skeleton.

The production method of the present invention is a method for producing a conductive sheet comprising a conductive substrate layer and a hydrogel layer formed of a silicone hydrogel having a polymer comprising a repeat unit (A) derived from a monomer represented by Formula (I) as a gel skeleton, the method comprising:

Step (ii): a step of bringing a silicone hydrogel precursor solution comprising a monomer represented by Formula (I) into contact with the conductive substrate layer; and
Step (iii): a step of polymerizing the silicone hydrogel.

EFFECTS OF THE INVENTION

The conductive sheet of the present invention can maintain adhesiveness and flexibility for a long period of time and is also superior in biocompatibility.

EMBODIMENTS OF THE INVENTION

<Conductive substrate layer>

In the present invention, as the conductive substrate layer, a structure formed of a conductive material that itself has conductivity, a structure formed of a material that exhibits conductivity by mixing a conductive material such as a carbon material or metal particles with a non-conductive material, a structure obtained by coating a structure formed of a non-conductive material such as resin with a conductive material, or the like may be chosen.

Examples of the conductive material include metal and carbon. Specific examples of the metal include gold, silver, stainless steel, titanium, copper, brass, bronze, tantalum, zirconium, nickel, and aluminum. From the viewpoint of being superior in corrosion resistance and economic efficiency, stainless steel or aluminum is more preferable, and aluminum is even more preferable.

Preferable examples of the material that exhibits conductivity by mixing a conductive material with a non-conductive material include a material prepared by kneading a carbon material with a resin. As the resin, it is preferable to use a resin selected from among polyester, polyamide, polyacrylic acid, polyurethane, polyolefin, polyvinyl alcohol, polyimide, polyvinylidene chloride, polyallylate, polyphenylene sulfide, polyether ether ketone, polytetrafluoroethylene, and polychlorotrifluoroethylene. From the viewpoint of being superior in flexibility, a resin selected from among polyester, polyamide, polyurethane, polyvinyl alcohol, and polyimide is more preferable, and polyester or polyimide is even more preferable. As the carbon material, a material selected from among carbon black, graphene, carbon nanotube, and fullerene is preferably used. From the viewpoint of ease of obtaining conductivity, a material selected from among carbon black, graphene, and carbon nanotubes is more preferable, and graphene or carbon nanotubes is even more preferable.

When a structure in which a structure made of a non-conductive material is coated with a conductive material is used, examples of the non-conductive material include the same resins as those described above. Examples of the conductive material coating the structure include an organic conductive material, an inorganic conductive material, and a carbon-based conductive material. As the organic conductive material, a polymeric conductive material is representative. Specific examples thereof include polyacetylene, polyaniline, polypyrrole, polythiophene, polyparaphenylene, and polyparaphenylenevinylene. Among them, from the viewpoint of ease of preparation, a material selected from among polyaniline, polypyrrole, and polythiophene is preferable, polyaniline or polythiophene is more preferable, and polythiophene is most preferable. In particular, it is preferable to use poly(3,4-ethylenedioxythiophene)/poly(4-styrenesulfonic acid) (abbreviated as PEDOT:PSS) as polythiophene from the viewpoint of being superior in handleability and conductivity. Examples of the inorganic conductive material include gold, silver, ITO, ATO, titanium oxide, copper, nickel, iron, and aluminum. Examples of the carbon-based conductive material include graphite, carbon black, graphene, and carbon nanotube. Graphene or carbon nanotube is more preferable from the viewpoint of being superior in coating property. Especially, graphene is preferable because it is easy to prepare its dispersion liquid.

In the application a living body, the conductive substrate layer preferably has air permeability in order to suppress stuffiness and prevent rash. The expression "have air permeability" as used herein means that the air permeability measured by a Frazier method is 0.2 $cm^3/cm^2 \cdot s$ or more. The air permeability is a value measured and calculated as air permeability Q [$cm^3/cm^2 \cdot s$] of the air permeability A method (Frazier type method) described in JIS L 1096 (2010). When the air permeability of the conductive substrate layer is 0.2 $cm^3/cm^2 \cdot s$ or more, a stuffy feeling is hardly felt, and the air permeability is more preferably 0.5 $cm^3/cm^2 \cdot s$ or more, and even more preferably 0.8 $cm^3/cm^2 \cdot s$ or more. Although the upper limit of the air permeability is not particularly limited, the air permeability is generally preferably 800 $cm^3/cm^2 \cdot s$ or less, more preferably 700 $cm^3/cm^2 \cdot s$ or less, and even more preferably 600 $cm^3/cm^2 \cdot s$ or less.

Examples of the structure constituting the conductive substrate layer having air permeability include a punched film, a fiber structure, and a foam. A punched film or a fiber structure is preferable in terms of flexibility.

The conductive substrate layer preferably has flexibility. In the present description, "have flexibility" means that the minimum bending radius is 12 $\mu m$ to 30 mm. When the minimum bending radius is 30 mm or less, for example, practical followability is exhibited even around a joint portion with large deformation. On the other hand, when the minimum bending radius is 12 $\mu m$ or more, the conductive substrate layer is hardly damaged during drawing or bending. Here, the minimum bending radius refers to a minimum curvature radius at which the substrate can be bent without irreversible

damage when the substrate is bent by 180 degrees. Here, the irreversible damage refers to a state of breaking, cracking, warping, creasing, or the like. When the flexibility of the conductive substrate layer is insufficient, the deformation of the conductive sheet cannot follow the movable portion of the human body, and not only causes detachment from the body surface but also causes damage due to delamination between the hydrogel layer and the conductive substrate layer. The same applies to a case where the conductive sheet is used with it being bonded to an object having a complicated shape made of concrete. The minimum bending radius of the conductive substrate layer is more preferably 20 mm or less, and even more preferably 10 mm or less. The minimum bending radius is more preferably 20 μm or more, and even more preferably 50 μm or more.

The thickness of the conductive substrate layer is chosen in consideration of flexibility and resistance to breakage. The thickness of the conductive substrate layer is preferably 10 mm or less, more preferably 5 mm or less, and even more preferably 2 mm or less. The thickness of the conductive substrate layer is preferably 1 μm or more, more preferably 5 μm or more, and even more preferably 10 μm or more. When the thickness of the conductive substrate layer is 10 mm or less, the conductive substrate layer easily follows the object to be bonded, and when the thickness is 1 μm or more, the conductive substrate layer is hardly damaged during drawing or bending.

A particularly preferable embodiment may be an embodiment in which a fiber structure mixed with a conductive material or a fiber structure coated with a conductive material is used as a conductive substrate layer in that stuffiness or rash is suppressed due to superior air permeability and a flexible conductive substrate layer is obtained. In particular, an embodiment in which a fiber structure containing fibers containing graphene is used as the conductive substrate layer is preferable.

Here, a fiber structure containing fibers containing graphene will be described. The graphene may be mixed in the fibers or coated on the surface of the fibers. From the viewpoint of being better in conductivity, a fiber structure in which the graphene is coated on the surface of fibers is more preferable. Hereinafter, the embodiment in which the fiber structure containing graphene is a fiber structure coated with graphene will be mainly described.

The fiber structure refers to a structure obtained by high-order processing monofilaments. Preferable examples of the fiber structure include woven fabric, knitted fabric, lace, and nonwoven fabric. Woven fabric, knitted fabric, or nonwoven fabric is more preferable in that they are superior in covering property with graphene, and woven fabric or nonwoven fabric is even more preferable in that the covering graphene is less likely to fall off during use.

The fiber constituting the fiber structure may be either a natural fiber or a chemical fiber. Examples of the natural fiber include vegetable fibers, animal fibers, and mineral fibers, and a vegetable fiber or an animal fiber is preferable from the viewpoint of being superior in handleability. As the vegetable fiber, cotton, hemp, or pulp is preferable, and cotton or pulp is more preferable. Paper using pulp or the like can also be preferably used. As the animal fiber, wool, silk, and cashmere are preferable, and wool or silk is more preferable. In particular, silk is preferable because its surface is superior in affinity with graphene and is easily coated. As the chemical fiber, a fiber selected from among polyester fibers, polyamide fibers, polyacrylic fibers, polyurethane fibers, polyolefin fibers, polyvinyl alcohol fibers, polyvinylidene chloride fibers, polyallylate fibers, polyphenylene sulfide fibers, polyether ether ketone fibers, polytetrafluoroethylene fibers, and polychlorotrifluoroethylene fibers is preferable. From the viewpoint of being superior in processability, a fiber selected from among polyester fibers, polyamide fibers, polyacrylic fibers, and polyurethane fibers is more preferable, and a polyester fiber or a polyamide fiber is more preferable.

The perimeter of the single fiber (in the present description, individual fibers constituting the fiber structure, including fibers constituting a spun yarn, a monofilament yarn, or a multifilament yarn spun from short fibers are referred to as "single fibers") constituting the fiber structure is preferably 30 μm or more and 300 μm or less. When the perimeter is less than 30 μm, the number of single fibers per unit volume increases and the number of contact points between single fibers increases, so that contact resistance tends to increase, leading to high resistance. On the other hand, when the perimeter of the single fiber exceeds 300 μm, the surface area becomes small, so that the amount of graphene attached per unit volume of the fiber decreases and the conductivity tends to decrease. The perimeter of the single fiber is preferably 30 μm or more, more preferably 45 μm or more, and even more preferably 60 μm or more. The perimeter of the single fiber is preferably 300 μm or less, more preferably 250 μm or less, and even more preferably 200 μm or less. The perimeter of a single fiber means the perimeter of a cross section perpendicular to the axial direction of the single fiber.

The single fiber diameter of the single fiber constituting the fiber structure is preferably 2 μm or more and 100 μm or less. When the single fiber diameter is smaller than 2 μm, the strength is insufficient and the durability of the fiber structure tends to lower. The single fiber diameter is preferably 5 μm or more, more preferably 10 μm or more, and even more preferably 20 μm or more. When the single fiber diameter is larger than 100 μm, the surface area is reduced, so that the amount of graphene attached per unit volume of the fiber tends to be reduced, leading to deterioration of conductivity. The single fiber diameter is preferably 80 μm or less, more preferably 60 μm or less, and even more preferably 40 μm or less.

The perimeter and the fiber diameter of a single fiber can be determined by photographing a cross section of the fiber structure cut with a microtome or the like using a scanning electron microscope, measuring the perimeter of a

cross section perpendicular to the axial direction of the fiber for 10 fibers, and averaging the measured values.

When woven fabric is used as the fiber structure, the mesh size of the woven fabric is preferably 1 μm or more and 200 μm or less. The mesh size is a value obtained as a square root of the area of a hole formed by being surrounded by warps and wefts in the woven fabric. Specifically, the mesh size of the woven fabric can be determined by observing the woven fabric surface with a scanning electron microscope at a magnification of 200 times, measuring the areas of holes formed by being surrounded by warps and wefts at 20 different locations, and determining the average value of the square roots thereof as the mesh size. When the mesh size is less than 1 μm, they tend to interfere with each other and the flexibility of the woven fabric tends to lower. When the mesh size is larger than 200 μm, yarns easily move, so that unevenness of the yarns tends to occur in a plane and the durability of the woven fabric tends to decrease. The mesh size of the woven fabric is preferably 3 μm or more, more preferably 5 μm or more, and even more preferably 10 μm or more. The mesh size of the woven fabric is preferably 150 μm or less, more preferably 100 μm or less, and even more preferably 50 μm or less.

The woven yarn pitch of the woven fabric is preferably 100 μm or more and 1500 μm or less for both the warp and the weft. The woven yarn pitch means a distance from the radial center of a woven yarn to the radial center of an adjacent woven yarn. When the woven yarn pitch is excessively small, adjacent woven yarns tend to interfere with each other and the flexibility of the woven fabric tends to lower. The woven yarn pitch is preferably 200 μm or more, more preferably 300 μm or more, and even more preferably 400 μm or more. When the woven yarn pitch is excessively large, the durability of the woven fabric, such as tear strength, tends to decrease. The woven yarn pitch is preferably 1200 μm or less, more preferably 900 μm or less, and even more preferably 600 μm or less. In a woven fabric, the pitch of the warps and the pitch of the wefts may be different.

The mesh size and pitch of the woven fabric, and the diameter (warp diameter and weft diameter) of the woven yarns constituting the woven fabric are in the relationship of "mesh size = pitch - diameter of woven yarn".

In a preferred embodiment, the fiber structure constituting the conductive substrate layer is constituted of a fiber containing graphene.

Graphene refers to a sheet of $sp^2$-bonded carbon atoms having a thickness of one atom (monolayer graphene) in a narrow sense, but in the present description, a sheet having a flaky form in which monolayer graphene is laminated is also referred to as graphene. In addition, similarly, graphene oxide is also referred to including those having a laminated flaky form.

In the present description, a material having an element ratio of oxygen atoms to carbon atoms (a degree of oxidation (O/C ratio)) measured by X-ray photoelectron spectroscopy (XPS) of more than 0.4 is called graphene oxide, and a material having an element ratio of 0.4 or less is called graphene. Accordingly, in the case of reduced graphene oxide obtained by reducing graphene oxide, one having a degree of oxidation (O/C ratio) of 0.4 or less is referred to as graphene.

Further, graphene and graphene oxide may be surfacetreated for the purpose of improving dispersibility, and in the present description, graphene and graphene oxide to which such a surface treatment agent is attached are also referred to as "graphene" or "graphene oxide".

In the case of coating the fiber surface, since graphene is in a thin sheet shape, it is easy to follow a complicated shape, and since individual fibers can be uniformly coated, conductivity is easily obtained. In addition, since graphene is insoluble in water, there is little concern that the signal acquisition performance is deteriorated due to elution by sweat or the like.

In the case of coating the fiber structure, the size of graphene in the direction parallel to the graphene layer is preferably 0.10 μm or more, more preferably 0.15 μm or more, and even more preferably 0.20 μm or more. Likewise, the size is preferably 10.00 μm or less, more preferably 8.00 μm or less, and even more preferably 6.00 μm or less. The size of graphene herein refers to the average of the longest diameter and the shortest diameter of the graphene in the direction parallel to the graphene layer. When the lower limit of the size of graphene in the direction parallel to the graphene layer is 0.10 μm or more, coating with graphene is less likely to be sparse, and thus conductivity is easily obtained. On the other hand, when the upper limit of the size of graphene in the direction parallel to the graphene layer is 10.00 μm or less, the dispersibility in the solvent is improved, and the coating on the fiber tends to be uniform.

The size of graphene in the direction parallel to the graphene layer can be calculated by collecting graphene from the conductive sheet, observing the graphene in an enlarged manner at a magnification of 1,500 to 50,000 times using an electron microscope such that the graphene appropriately fits in the field of view, measuring the length (major axis) of the longest portion and the length (minor axis) of the shortest portion in the direction parallel to the graphene layer for each of 10 randomly selected graphenes, and determining the arithmetic average value of the numerical values obtained by (major axis + minor axis)/2.

The size of graphene in the direction parallel to the graphene layer can be easily adjusted to the above-described range by refining graphene oxide or reduced graphene. Commercially available graphene oxide or graphene having a desired size may be used.

The average thickness of graphene is preferably 100 nm or less, more preferably 50 nm or less, and even more

preferably 20 nm or less. Likewise, the average thickness is preferably 1 nm or more, more preferably 1.5 nm or more, and even more preferably 2 nm or more. When the average thickness of graphene is 100 nm or less, the flexibility of the graphene can be maintained and the graphene tends to easily follow the fiber surface. On the other hand, when the average thickness of the graphene is 1 nm or more, the conductivity tends to be easily enhanced.

The average thickness of graphene can be calculated by collecting graphene by mechanically peeling the graphene from the conductive sheet, observing the graphene with an atomic force microscope in an enlarged manner in a square field of view of about 1 to 10 μm on each side such that the graphene can be appropriately observed, measuring the thickness of each of 10 randomly selected graphenes, and determining the arithmetic average value thereof. The thickness of each graphene is an arithmetic average value of measured values of thicknesses at five randomly selected points for each graphene.

When it is difficult to mechanically collect graphene, for a fiber structure covered with graphene, the fiber structure is dissolved or dispersed using an alkaline bath or the like, and then the remaining graphene material can be measured in the same manner after being separated.

The degree of oxidation (O/C ratio) of graphene is preferably 0.05 or more, more preferably 0.07 or more, even more preferably 0.09 or more, and further preferably 0.10 or more. The degree of oxidation is preferably 0.35 or less, more preferably 0.30 or less, even more preferably 0.20 or less, and further preferably 0.15 or less. When the degree of oxidation (O/C ratio) is 0.05 or more, graphene is likely to be uniformly distributed on the fiber. On the other hand, when the degree of oxidation (O/C ratio) is 0.35 or less, the π-electron conjugated structure is restored by reduction of graphene, so that conductivity is obtained.

When the thickness of the graphene coating layer on the fiber constituting the fiber structure is 1 nm or more, good conductivity is obtained, and when the thickness is 100 nm or less, the ease of stretching and bending of the conductive substrate layer is hardly affected. The thickness of the graphene coating layer on the fiber is preferably 1 nm or more, more preferably 2 nm or more, even more preferably 5 nm or more, and further preferably 10 nm or more. The thickness of the graphene coating layer is preferably 100 nm or less, more preferably 90 nm or less, and even more preferably 80 nm or less. The thickness of the graphene coating layer on the fiber can be determined by observing a cross section of the fiber cut with a microtome or the like with a scanning electron microscope, and determining the thickness from the average value of the measurement results at 10 points.

<Hydrogel layer>

The conductive sheet of the present invention has a hydrogel layer made of silicone hydrogel in addition to the conductive substrate layer described above. In the present description, a substance in which water or the like is supported on a silicone-based polymer is basically referred to as "silicone hydrogel", but for simplification of explanation, "silicone hydrogel" may be used as a term indicating a polymer itself constituting the gel skeleton of a silicone hydrogel.

The silicone hydrogel exhibits conductivity by containing water. The silicone hydrogel preferably has a water content of 10% by weight or more and less than 60% by weight. In the present description, the water content refers to a value determined by measuring the weight (W1) of the silicone hydrogel in a hydrous state and the weight (W2) thereof in a dry state, and calculating the water content by the following formula.

$$\text{Water content (\%)} = (W1 - W2) / W1 \times 100.$$

Here, the term "at the time of containing water" means a state in which the silicone hydrogel has been immersed in distilled water at room temperature (25°C) for 24 hours or more. A test piece is taken out from the distilled water, the distilled water on the surface of the test piece is lightly wiped with a cloth made of a material to which dirt such as dust does not adhere, and then a physical property value at the time of containing water is measured within 1 minute after the test piece was taken out from the distilled water. The dry state means a state after a silicone hydrogel in a hydrous state has been dried in a vacuum dryer at 40°C for not less than 16 hours. The measurement of a physical property value in a dry state is performed within 1 minute after the test piece is taken out from a drying device.

When the water content of the silicone hydrogel is 10% by weight or more, the conductivity is high, and when the water content is less than 60% by weight, a shape change accompanying drying is small. The water content is more preferably 15% by weight or more, and even more preferably 20% by weight or more. The water content is more preferably 55% by weight or less, and even more preferably 50% by weight or less.

The hydrogel layer preferably contains 50% by weight or more, more preferably 60% by weight or more, even more preferably 70% by weight or more, and further preferably 80% by weight or more of the polymer constituting the gel skeleton of the silicone hydrogel, in a dry state. Here, the "polymer constituting the gel skeleton of a silicone hydrogel" is a polymer forming a network structure of the silicone hydrogel. In the present invention, the polymer constituting the gel skeleton of a silicone hydrogel is a polymer containing a repeat unit (A) derived from a monomer represented by the

following Formula (I).

Here, the content (% by weight) of the polymer constituting the gel skeleton of the silicone hydrogel in a dry state in the hydrogel layer is measured as follows. The hydrogel layer extracted from the conductive sheet is dried in a vacuum dryer at 40°C for 16 hours or more, and the weighing value (E1) immediately after drying is measured. Next, the dried hydrogel layer is extracted in acetone at 37°C for 72 hours to remove residual monomers and components such as a wetting agent other than the polymer constituting the gel skeleton, and then dried in a vacuum dryer at 40°C for 16 hours or more, and a weighing value (E2) immediately after drying is measured. The content (% by weight) of the polymer constituting the gel skeleton of the silicone hydrogel in a dry state in the hydrogel layer can be determined from E2/E1 × 100.

When the conductive sheet of the present invention is applied to a living body, the content of silicon atoms in the silicone hydrogel for use in the present invention based on the total weight in a dry state (hereinafter, simply referred to as "silicon atom content") is preferably in a range of 5% by weight or more and 30% by weight or less. When the silicon atom content is 5% by weight or more, oxygen permeability is easily obtained, and when the silicon atom content is 30% by weight or less, skin adhesiveness is hardly impaired. The silicon atom content is more preferably 7% by weight or more, and even more preferably 10% by weight or more. The silicon atom content is more preferably 28% by weight or less, and even more preferably 25% by weight or less.

When m kinds (m is an integer of 1 or more) of silicone monomers (monomers containing a polymerizable group and a siloxanyl group) are used for forming the silicone hydrogel, the silicon atom content refers to a value calculated from the following formula. The siloxanyl group refers to any group having at least one Si-O-Si linkage.

$$\text{Content (\%) of silicon atoms in the total weight of silicone hydrogel in a dry state} = \Sigma\{Si_m \times C_m\} \times 100$$

$Si_m$ is the weight ratio of the silicon atoms to the molecular weight of the mth silicone monomer. $C_m$ represents the weight ratio of the mth silicone monomer to the dry weight of the silicone hydrogel.

As one example, a description is given using a silicone hydrogel obtained by polymerizing a monomer composition composed of 25% by weight of 3-[tris(trimethylsiloxy)silyl]propyl methacrylate (abbreviation: TRIS), 24% by weight of 3-(trimethoxysilyl)propyl methacrylate (abbreviation: TMSM), 49% by weight of N,N-dimethylacrylamide, and 2% by weight of ethylene glycol dimethacrylate. The weight of the silicon atoms is calculated as 28.09 (g/mol). In this example, there are two kinds of silicone monomers, and a calculation example is shown where the first kind is TRIS and the second kind is TMSM. Since TRIS has 4 silicon atoms with respect to the molecular weight of 422.82, the weight ratio $Si_1$ of silicon atoms to the molecular weight of TRIS can be calculated as $Si_1$ = 28.09 × 4/422. 82 = 0.266, and $C_1$ is 0.25. Since TMSM has one silicon atom with respect to the molecular weight of 248.35, the weight ratio $Si_2$ of silicon atoms to the molecular weight of TMSM can be calculated as $Si_2$ = 28.09 × 1/248. 35 = 0.113, and $C_2$ is 0.24. Other monomers do not contain silicon atoms. Thus, the silicon atom content (%) of the silicone hydrogel can be calculated as (0.266 × 0.25 + 0.113 × 0.24) × 100 = 9.362.

The silicon atom content can be experimentally measured by ICP emission spectrometry. Specifically, the measurement is performed according to the following procedure. A sample of the silicone hydrogel is washed with RO water to remove salts and stains, then a sample (4 to 5 mg) weighed in a dry state is weighed in a platinum crucible, sulfuric acid is added thereto, and the sample is incinerated by heating with a hot plate and a burner. Such an incinerated product is melted with sodium carbonate, water is added thereto, and the mixture is heated and dissolved, then nitric acid is added thereto, and the volume is fixed with water. For this solution, the amount of silicon atoms is measured by ICP emission spectrometry, and the content in the sample is determined.

The polymer constituting the gel skeleton of the silicone hydrogel for use in the present invention comprises a repeat unit (A) derived from a monomer represented by Formula (I).

[Chemical Formula 1]

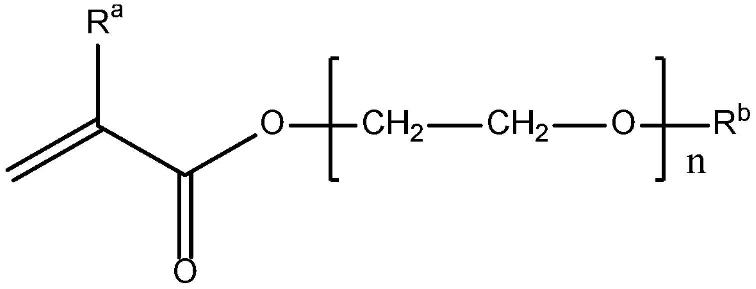

In Formula (I), $R^a$ represents a hydrogen atom or a methyl group, and $R^b$ represents an organic group having 1 to 15 carbon atoms. n represents an integer in a range of 1 to 40.

Here, the "repeat unit derived from a monomer" will be described. In the present invention, the "repeat unit derived from a monomer" means a structural unit in a polymer that is generated by the change of a radical polymerizable functional group due to a polymerization reaction when a radical polymerizable monomer is polymerized and that corresponds to the structure of the monomer. That is, the "repeat unit derived from a monomer" is a structural unit represented by the following Formula (y) which is generated by the change of a radically polymerizable functional group due to a polymerization reaction when a radically polymerizable monomer represented by the following Formula (x) is polymerized.

[Chemical Formula 2]

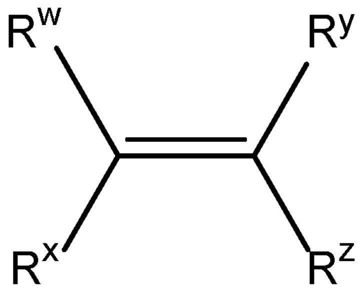

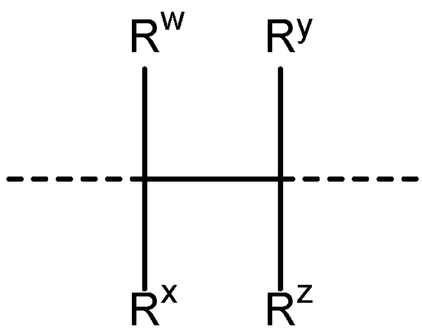

In the above Formulae (x) and (y) , $R^w$, $R^x$, $R^y$, and $R^z$ may each independently be any group as long as the monomer represented by Formula (x) can be a monomer having radical polymerizability.

Such a repeat unit (A) is hydrophilic, but has no functional groups capable of hydrogen bonding with each other, and thus has a property that interaction between functional groups due to hydrogen bonding is small and the repeat unit

(A) is hardly stiffened at low water content. In particular, since it has a structure having a (poly)ethylene glycol chain, it is useful because it has a high effect of decreasing the elastic modulus at low water content. The repeat unit (A) has a glass transition temperature of room temperature or lower when the glass transition temperature of a homopolymer of the monomer represented by Formula (I) is measured, and therefore the tensile elastic modulus in a dry state is kept low.

$R^a$ in Formula (I) is a hydrogen atom or a methyl group from the viewpoint of being superior in polymerizability and in easy availability, and is more preferably a hydrogen atom because superior flexibility tends to be obtained.

n in Formula (I) is an integer in the range of 1 to 40. When n is 40 or less, the compatibility with other monomers hardly deteriorate, and the crosslinking density of the entire silicone hydrogel lowers, so that the silicone hydrogel is less likely to be torn. n is more preferably 25 or less, even more preferably 12 or less, and further preferably 10 or less. n may have a distribution. Having a distribution refers to a state in which molecules differing in the number (here, n) representing the repetition of a certain structural unit are mixed. When n has a distribution, n is determined based on the number average molecular weight of the monomer represented by Formula (I).

$R^b$ in Formula (I) is an organic group having 1 to 15 carbon atoms. $R^b$ is preferably an alkyl group having 1 to 15 carbon atoms, more preferably a group selected from among an ethylhexyl group, a butyl group, a propyl group, an ethyl group, and a methyl group, even more preferably a group selected from among a propyl group, an ethyl group, and a methyl group, and further preferably an ethyl group or a methyl group, from the viewpoint of being less likely to cause an interaction such as a hydrogen bond and being less likely to increase the tensile elastic modulus in a dry state.

$R^b$ may be, for example, a group having a cyclic structure such as a furfuryl group or a crown ether.

$R^b$ may be a fluorine-substituted alkyl group. Among the monomers represented by Formula (I), examples of such monomers include 2-(2,2,2-trifluoroethoxy)ethyl acrylate and 2-(2,2,3,3,4,4,-heptafluorobutoxy)ethyl acrylate.

When the conductive sheet of the present invention is applied to a living body, the silicone hydrogel preferably contains the repeat unit (A) in a range of 10% by weight to 70% by weight with respect to the total weight of the silicone hydrogel in a dry state. When the content of the repeat unit (A) is 10% by weight or more, the tensile elastic modulus in a dry state is not excessively high. When the content is 70% by weight or less, the adhesiveness to the skin is hardly to be insufficient. The content of the repeat unit (A) is more preferably 10% by weight or more, even more preferably 20% by weight or more, and further preferably 30% by weight or more. The content of the repeat unit (A) is more preferably 70% by weight or less, even more preferably 60% by weight or less, and further preferably 50% by weight or less.

As the silicone monomer, silicone (meth)acrylate or silicone (meth)acrylamide can be preferably used. Silicone (meth)acrylate is preferably used because it is relatively easily available. Here, the silicone (meth)acrylate refers to a monomer containing a (meth)acryloyloxy group and a siloxanyl group. The silicone (meth)acrylamide refers to a monomer containing a (meth)acrylamide group and a siloxanyl group. In the present description, the term "(meth)acrylate" represents both methacrylate and acrylate. The same applies to terms such as "(meth)acrylamide" and "(meth)acryloyl". The term "(meth)acrylate" as used herein refers to a (meth)acrylic acid ester.

The silicone monomer to be used in the present invention may be linear or branched. When a silicon atom bonded to an organic group having a polymerizable group is taken as a starting point, the linear silicone monomer refers to a structure in which, when a line along a siloxanyl bond (a bond formed by repeating -Si-O-) of silicone is drawn, the line is formed in a single linear shape with no branches. On the other hand, the branched silicone monomer refers to a structure in which, when a line along a siloxanyl bond is drawn starting from a silicon atom bonded to an organic group having a polymerizable group, the line extends in two or more directions and/or a structure in which the line has at least one branch and cannot be represented as one line. As a representative example of such a branched silicone monomer, 3-[tris(trimethylsiloxy)silyl]propyl methacrylate (abbreviation: TRIS) is known.

When the conductive sheet of the present invention is applied to a living body, the polymer constituting the gel skeleton of the silicone hydrogel preferably further comprises a repeat unit (B) derived from a monofunctional silicone monomer. The term "monofunctional" means having only one radical polymerizable functional group (for example, a (meth)acryloyloxy group) in the molecule. By containing such a repeat unit (B), superior oxygen permeability, flexibility, and bendability can be imparted.

In particular, as the monofunctional silicone monomer, a monomer represented by the following Formula (p) is preferable.

[Chemical Formula 3]

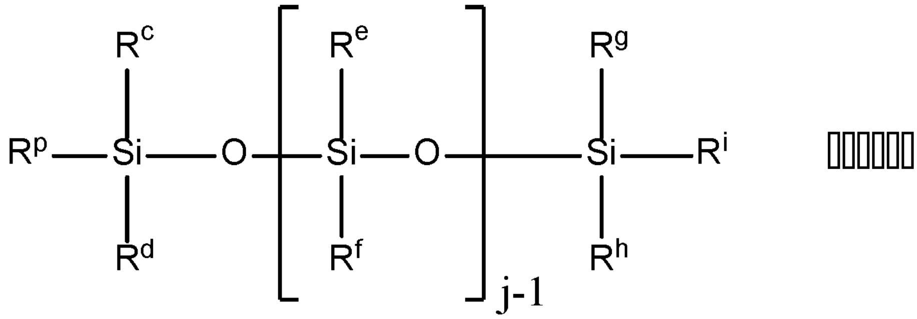

In Formula (p), RP represents an alkyl group having a (meth)acryloyloxy group or a (meth)acrylamide group. $R^c$ to $R^i$ each represent a group containing no silicon atoms, and j represents an integer of 1 or more.

The polymer constituting the gel skeleton of the silicone hydrogel for use in the present invention preferably comprises the repeat unit (B) derived from the monofunctional silicone monomer in a range of 10% by weight or more and 70% by weight or less with respect to the total weight of the silicone hydrogel in a dry state. When the content of the repeat unit (B) is 10% by weight or more, oxygen permeability and flexibility are enhanced. When the content is 70% by weight or less, the adhesiveness to the skin is enhanced. Likewise, the content of the repeat unit (B) is more preferably 20% by weight or more, and even more preferably 30% by weight or more. The content of the repeat unit (B) is more preferably 60% by weight or less, and even more preferably 50% by weight or less.

As the monofunctional silicone monomer, silicone (meth)acrylate or silicone (meth)acrylamide having a linear siloxanyl group is preferably used. Particularly preferred examples include a monomer represented by the following Formula (a).

[Chemical Formula 4]

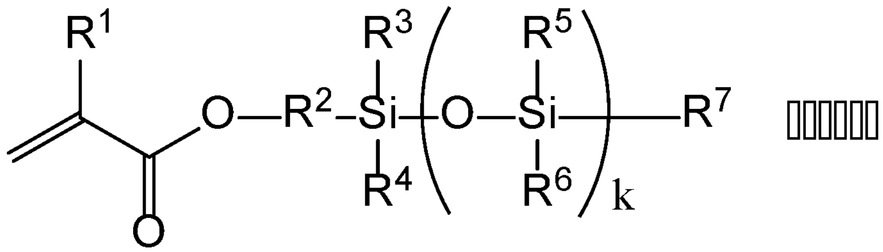

In Formula (a), $R^1$ represents a hydrogen atom or a methyl group. X represents an oxygen atom or NH. $R^2$ represents a divalent organic group having 1 to 20 carbon atoms. $R^3$ to $R^6$ each independently represent an alkyl group having 1 to 20 carbon atoms or an aryl group having 6 to 20 carbon atoms. $R^7$ represents an alkyl group having 1 to 20 carbon atoms or an aryl group having 6 to 20 carbon atoms. k represents an integer of 1 to 200 which may have a distribution.

In Formula (a), $R^1$ is a hydrogen atom or a methyl group, and among these, a methyl group is more preferable in that a monomer is easily available.

In Formula (a), X is an oxygen atom or NH, but an oxygen atom is preferable in that a monomer is easily available, and NH is preferable in that the polymerization rate can be improved.

In Formula (a), $R^2$ is a divalent organic group having 1 to 20 carbon atoms, and examples thereof include alkylene groups such as a methylene group, an ethylene group, a propylene group, a butylene group, a pentylene group, an octylene group, a decylene group, a dodecylene group, and an octadecylene group; and arylene groups such as phenylene groups and naphthylene groups. These alkylene and arylene groups may be either linear or branched. When the number of carbon atoms of the divalent organic group is 20 or less, compatibility with other monomers is hardly reduced, and when the number of carbon atoms is 1 or more, the flexibility of the silicone hydrogel to be obtained is hardly reduced. The number of the carbon atoms in $R^2$ is more preferably in the range of 1 to 12, and even more preferably in the range of 2 to 8. In the divalent organic group, an alkylene unit may be replaced by an oxygen atom or a sulfur atom, and a hydrogen atom adjacent to carbon may be replaced by a hydroxyl group, an amino group, or a halogen atom. Examples of suitable substituents in the case where the divalent organic group is substituted include substituents

such as a hydroxyl group, a carboxyl group, a sulfonic acid group, a phosphoric acid group, an ester, an ether, and an amide, and combinations thereof. Among them, a hydroxyl group, an ester, an ether, and an amide are preferable in that the decomposition of the silicone moiety hardly occurs, and an ether is more preferable in that the stability of the silicone hydrogel is enhanced.

More suitable examples of $R^2$ include an ethylene group, a propylene group, a butylene group, and divalent organic groups represented by the following Formulas (a1) to (a4) .

$-CH_2CH\ (OH)\ CH_2-$ (a1)

$-CH_2CH(OH)CH_2OCH_2CH_2CH_2-$ (a2)

$-CH_2CH_2OCH_2CH_2CH_2-$ (a3)

$-CH_2CH_2OCH_2CH_2OCH_2CH_2CH_2-$ (a4)

Among them, a propylene group and divalent organic groups represented by Formulas (a1) to (a4) are preferable, and a propylene group or a divalent organic group represented by Formula (a2) is more preferable.

In Formula (a), $R^3$ to $R^6$ each independently represent an optionally substituted alkyl group having 1 to 20 carbon atoms or an optionally substituted aryl group having 6 to 20 carbon atoms. Examples thereof include a methyl group, an ethyl group, a n-propyl group, an isopropyl group, a n-butyl group, a s-butyl group, a t-butyl group, a n-pentyl group, an isopentyl group, a s-pentyl group, a neopentyl group, a hexyl group, a heptyl group, an octyl group, a nonyl group, a decyl group, a dodecyl group, an eicosyl group, a phenyl group, and a naphthyl group. These alkyl and aryl groups may be either linear or branched. When $R^3$ to $R^6$ each are an alkyl group having 1 or more and 20 or less carbon atoms or an optionally substituted aryl group having 6 or more and 20 or less carbon atoms, the silicone content is relatively reduced, and it is possible to suppress decrease in oxygen permeability of the silicone hydrogel to be obtained. The number of the carbon atoms of $R^3$ to $R^6$ is more preferably 1 to 12, and even more preferably 1 to 6.

In Formula (a), $R^7$ represents an optionally substituted alkyl group having 1 to 20 carbon atoms or an optionally substituted aryl group having 6 to 20 carbon atoms. When the number of the carbon atoms of $R^7$ is 1 or more, it is possible to suppress the polysiloxane chain from being easily hydrolyzed, and when the number of the carbon atoms of $R^7$ is 20 or less, it is possible to prevent the oxygen permeability of the silicone hydrogel from being lowered. An alkyl group having 1 to 10 carbon atoms or an aryl group having 6 to 10 carbon atoms is more preferable, an alkyl group having 1 to 6 carbon atoms is even more preferable, and an alkyl group having 1 to 4 carbon atoms is further preferable. Suitable examples of the alkyl group having 1 to 20 carbon atoms and the aryl group having 6 to 20 carbon atoms include a methyl group, an ethyl group, a n-propyl group, an isopropyl group, a n-butyl group, a s-butyl group, a t-butyl group, a n-pentyl group, an isopentyl group, a s-pentyl group, a neopentyl group, a hexyl group, a heptyl group, an octyl group, a nonyl group, a decyl group, a dodecyl group, an eicosyl group, a phenyl group, and a naphthyl group. These alkyl and aryl groups may be either linear or branched.

In Formula (a), k represents an integer of 1 or more and 200 or less which may have a distribution. When k is 200 or less, compatibility with other monomers hardly decreases, and when k is 1 or more, oxygen permeability and flexibility can be obtained. k is preferably 2 or more, and more preferably 3 or more. In addition, k is preferably 200 or less, more preferably 100 or less, even more preferably 50 or less, and further preferably 20 or less. When k has a distribution, k is determined based on the number average molecular weight of the monofunctional silicone monomer.

The polymer constituting the gel skeleton of the silicone hydrogel for use in the present invention preferably further comprises a repeat unit (C) derived from a monomer containing an amide structure. Such a repeat unit (C) is used for imparting a preferable water content and adhesiveness to the silicone hydrogel.

In the present invention, the monomer containing an amide structure refers to a compound in which a radical polymerizable functional group is bonded to a structure represented by the following Formula (q). Examples thereof include an amide compound, an imide compound, a urea compound, and derivatives thereof.

[Chemical Formula 5]

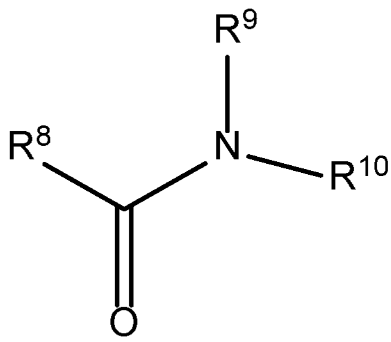

In Formula (q), the radical polymerizable functional group is bonded to any position of $R^8$ to $R^{10}$ directly or via a divalent organic group having 1 to 20 carbon atoms. When no radical polymerizable functional group is bonded, $R^8$ to $R^{10}$ each independently represent a hydrogen atom, a hydroxyl group, or an optionally substituted organic group having 1 to 20 carbon atoms, and may be either linear or branched. $R^8$ and $R^9$ or $R^{10}$ may be linked to each other to form a ring. When the number of the carbon atoms of the organic group is 20 or less, compatibility with a silicone monomer tends to be easily obtained. The number of the carbon atoms of the organic group is more preferably 1 to 12, and even more preferably 2 to 8.

The amide structure is less likely to be hydrolyzed than an ester bond or the like, and can exhibit superior durability. It can be expected that by containing the amide structure, decomposition, elimination, transformation, and the like of a part of the polymer structure are suppressed even under a severe environment such as high temperature and high humidity at the time of steam sterilization. In addition, it is preferable because it has a structure that gives superior water content and adhesiveness.

The repeat unit (C) preferably contains no ionic functional group. When an ionic functional group is contained, the pH of the silicone hydrogel is changed by sweat or the like, which may cause rash or the degradation of the gel.

The polymer constituting the gel skeleton of the silicone hydrogel for use in the present invention preferably contains the repeat unit (C) in a range of 10% by weight or more and 50% by weight or less with respect to the total weight of the silicone hydrogel in a dry state. When the content of the repeat unit (C) is 10% by weight or more, the water content of the silicone hydrogel is high, and when the content is 50% by weight or less, decrease in gel strength caused by excessive swelling and dimensional change associated with drying hardly occur. The content of the repeat unit (C) is more preferably 20% by weight or more, and even more preferably 30% by weight or more. The content of the repeat unit (C) is more preferably 45% by weight or less, and even more preferably 40% by weight or less.

Examples of monomers suitable for forming the repeat unit (C) include (meth)acrylamide, N-vinylcarboxylic acid amide, cyclic N-vinyllactam, cyclic N-vinylpyridine, N-vinylimidazole, N-vinylurea, and derivatives thereof. (Meth)acrylamide and derivatives thereof are more preferable in that they are superior in hydrophilicity.

Suitable specific examples of the monomer from which the repeat unit (C) can be obtained include acrylamide, N-methylacrylamide, N-ethylacrylamide, N-isopropylacrylamide, N,N-dimethylacrylamide, N,N-diethylacrylamide, N,N-diisopropylacrylamide, acryloylmorpholine, diacetone acrylamide, N-vinylformamide, N-vinylacetamide, N-vinyl-N-methylacetamide, N-vinylpyrrolidone, N-vinyl-2-piperidone, N-vinyl-2-caprolactam, N-vinyl-3-methyl-2-caprolactam, N-vinyl-4-methyl -2 caprolactam, 2-ethyloxazoline, N-(2-hydroxypropyl) (meth)acrylamide, N-(2-hydroxyethyl) (meth)acrylamide, N-vinyl-2-methylpropionamide, N-vinyl-N,N'-dimethylurea, dimethylaminopropyl (meth)acrylamide, dimethylaminoethyl (meth)acrylamide, dimethylaminopropyl (meth)acrylate, and dimethylaminoethyl (meth)acrylate.

Among these, a monomer selected from among N-methylacrylamide, N,N-dimethylacrylamide, N-vinylpyrrolidone, N-vinylcaprolactam, N-vinylacetamide, and N-vinyl-N-methylacetamide is more preferable in that it is superior in compatibility with other monomers. These may be used singly or two or more of them may be used in combination.

The polymer constituting the gel skeleton of the silicone hydrogel for use in the present invention may further comprise a repeat unit derived from a polyfunctional monomer having two or more polymerizable groups (crosslinking monomer). Preferred examples of the polyfunctional monomer having two or more polymerizable groups include ethylene glycol di(meth)acrylate, diethylene glycol di(meth)acrylate, triethylene glycol di(meth)acrylate, tetraethylene glycol di(meth)acrylate, neopentyl glycol di(meth)acrylate, tetraethylene glycol di(meth)acrylate, 1,3-bis(3-(meth)acryloyloxypropyl)-1,1,3,3-tetramethyl-disiloxane, 1,3-bis(3-(meth)acryloyloxypropyl)-1,1,3,3-tetrakis(trimethylsiloxy)disiloxane, polydimethylsiloxanes having (meth)acryloyloxy groups at both terminals such as X-22-164A manufactured by Shin-Etsu Chemical Co., Ltd. and "Silaplane" (registered trademark) FM7711 manufactured by JNC Corporation, di- or multifunctional (meth)acrylates such as glyceryl tri(meth)acrylate, pentaerythritol tetra(meth)acrylate, and trimethylolpropane

tri(meth)acrylate; and bisacrylamides such as N,N'-methylenebisacrylamide, N,N'-ethylenebisacrylamide, and N,N'-propylenebisacrylamide.

Among them, bifunctional (meth)acrylates are more preferable in that a silicone hydrogel superior in flexibility is easily obtained, and silicone-based di(meth)acrylates such as 1,3-bis(3-(meth)acryloyloxypropyl)-1,1,3,3-tetramethyldisiloxane, 1,3-bis(3-(meth)acryloyloxypropyl)-1,1,3,3-tetrakis(trimethylsiloxy)disiloxane, and polydimethylsiloxanes having (meth)acryloyloxy groups at both terminals such as X-22-164A, X-22-164B, and X-22-164C manufactured by Shin-Etsu Chemical Co., Ltd. and "Silaplane" (registered trademark) FM7711 and FM7721 manufactured by JNC Corporation are more preferable.

When the content of a repeat unit derived from a polyfunctional monomer having two or more polymerizable groups is 25% by weight or less based on the total weight of the silicone hydrogel in a dry state, the silicone hydrogel is less likely to be brittle, and when the content is 1% by weight or more, the tensile strength is high. The content of the repeat unit derived from the polyfunctional monomer is more preferably 5% by weight or more, and even more preferably 10% by weight or more. The content is more preferably 20% by weight or less, and even more preferably 15% by weight or less.

The silicone hydrogel layer for use in the present invention may contain an electrolyte for assisting conductivity. As such an electrolyte, for example, the following inorganic or organic acids and bases can be used. Examples of the bases include an alkali metal, an alkaline earth metal, ammonium, and pyridinium. Examples of the acids include inorganic acids such as boric acid, halogens, hypochlorous acid, chlorous acid, chloric acid, perchloric acid, sulfuric acid, nitric acid, and phosphoric acid; acetic acid, benzoic acid, lactic acid, phthalic acid, succinic acid, adipic acid, citric acid, tartaric acid, sulfonic acids, and amino acids. Further, examples of a polymeric acid include poly(meth)acrylic acid and polystyrene sulfonic acid, and examples of a polymeric base include polyallylamine and polyethyleneimine. These compounds may be used singly or two or more of them may be used in combination.

The conductive sheet of the present invention comprises a conductive substrate layer and a hydrogel layer. In the conductive sheet, the conductive substrate layer may be laminated on the hydrogel layer, or the conductive substrate layer may be embedded in the hydrogel layer. There is little possibility that the conductive substrate layer and the hydrogel layer are peeled off due to entanglement of the conductive substrate layer with a silicone hydrogel and the mechanical strength of the conductive sheet is enhanced. Therefore, an embodiment in which the conductive substrate layer is embedded in the hydrogel layer is more preferable. The hydrogel layer may be formed only on a part of the conductive substrate layer, and conversely, the conductive substrate layer may be formed only on a part of the hydrogel layer. In particular, especially when the conductive substrate layer has air permeability, entanglement of the silicone hydrogel with the substrate layer becomes denser, so that the mechanical strength of the conductive sheet can be further enhanced.

In the conductive sheet of the present invention, the impedance of the conductive substrate layer is preferably $10^{-3}$ Ω or more and $10^{7}$ Ω or less in the entire range of 0.1 Hz to 1000 Hz when measured in accordance with the method described in Examples. An impedance of $10^{-3}$ Ω or more is preferable because the conductive sheet is hardly affected by noise, and an impedance of $10^{7}$ Ω or less is preferable because it is easy to acquire an electric signal. The impedance is more preferably $10^{-2}$ Ω or more, and even more preferably $10^{-1}$ Ω or more. The impedance is more preferably $10^{6}$ Ω or less, and even more preferably $10^{5}$ Ω or less.

In order for the conductive sheet of the present invention to exhibit adhesiveness to an article to be bonded, the surface tack strength of the hydrogel layer in the conductive sheet in a dry state is preferably 1 N or more and 30 N or less. When the surface tack strength is 1 N or more, the conductive sheet hardly detaches from the surface to be bonded, and when the surface tack strength is 30 N or less, damage to the gel itself at the time of detachment can be reduced. The surface tack strength is preferably 1 N or more, more preferably 1.5 N or more, and even more preferably 2 N or more when measured in accordance with the method described in Examples. The surface tack strength is preferably 30 N or less, more preferably 25 N or less, and even more preferably 20 N or less.

When the surface tack strength of the conductive sheet in a dry state is within the above preferable range, adhesiveness can be maintained even when the conductive sheet is dried. That is, even when the conductive sheet is used for a long period of time, adhesiveness can be maintained.

In the conductive sheet of the present invention, a surface that is not to be in contact with an article to be bonded may be coated with a non-conductive material. When coating with such a non-conductive material is performed, for example, in the case of using the conductive material as an electrode, it is possible to prevent a signal from failing to be normally acquired due to a plurality of electrodes' short-circuiting in an environment where a large amount of perspiration or rain is applied. The non-conductive material is preferably a material that does not impair the permeability of moisture and gas of the conductive sheet of the present invention. Suitable examples of the non-conductive material include a resin film, a resin foam, a fiber structure, and a waterproof moisture-permeable material using a silicone elastomer. Such a non-conductive material may be coated with an adhesive layer interposed therebetween.

Another aspect of the present invention is a conductive sheet comprising a conductive substrate layer and a hydrogel layer comprising a silicone hydrogel, wherein the hydrogel layer in a dry state has a tensile elastic modulus in the range of 0.1 MPa or more and 3.5 MPa or less. When the tensile elastic modulus of the silicone hydrogel in a dry

state is 0.1 MPa or more, the hydrogel is hardly broken, and when the tensile elastic modulus is 3.5 MPa or less, the gel is hardly hardened even when dried, and adhesiveness is likely to be obtained. The tensile elastic modulus in a dry state is preferably 0.1 MPa or more, more preferably 0.3 MPa or more, even more preferably 0.4 MPa or more, and further preferably 0.5 MPa or more. The tensile elastic modulus in a dry state is preferably 3.5 MPa or less, more preferably 3.0 MPa or less, and even more preferably 2.5 MPa or less. The tensile elastic modulus in a dry state can be measured in accordance with the method described in Examples described later after extracting the silicone hydrogel from the structure. Such a preferable tensile elastic modulus can be achieved by the silicone hydrogel of the present invention having the above preferred composition.

When the tensile elastic modulus of the hydrogel layer in a dry state is within the above preferable range, flexibility can be maintained even when the conductive sheet is dried. That is, even when the conductive sheet is used for a long period of time, flexibility can be maintained.

The specification of the conductive substrate layer is the same as those described above.

<Method for producing conductive sheet>

Hereinafter, a method for producing the conductive sheet of the present invention will be described.

The conductive sheet of the present invention can be produced by a production method comprising:

Step (ii): a step of bringing a silicone hydrogel precursor solution comprising a monomer represented by Formula (I) into contact with the conductive substrate layer; and
Step (iii): a step of polymerizing the polymerization stock solution.

As described above, the conductive substrate layer is preferably a fiber structure in which the fiber surface is coated with graphene.

The fiber structure containing graphene may be formed by high-order processing monofilaments coated with graphene or may be formed by high-order processing monofilaments to form a fiber structure and then coating the fiber structure with graphene. However, from the viewpoint of ease of operation, it is preferable to form a fiber structure and then coat the fiber structure with graphene.

That is, the production method preferably has, before Step (ii),

Step (i): a step of coating the fiber structure with graphene to obtain a conductive substrate layer.

In Step (i), the fiber structure may be coated using a dispersion liquid of graphene, or alternatively, the conductive substrate layer may be obtained by coating the fiber structure using a dispersion liquid of graphene oxide and then subjecting the fiber structure to a reduction treatment to convert the graphene oxide into graphene. Graphene oxide is better in dispersibility in a solvent and is better in covering property with respect to a fiber structure, and therefore it is preferable to obtain a fiber structure comprising a fiber coated with graphene prepared by coating the fiber with a dispersion liquid of graphene oxide and then subjecting the fiber to a reduction treatment.

When a graphene oxide dispersion liquid is used as a dispersion liquid for coating, the degree of oxidation (O/C ratio) of the graphene oxide to be used is preferably 0.5 or more, more preferably 0.52 or more, and even more preferably 0.55 or more. The degree of oxidation (O/C ratio) is preferably 0.8 or less, more preferably 0.78 or less, and even more preferably 0.75 or less. When the degree of oxidation (O/C ratio) of graphene oxide is 0.5 or more, dispersibility in a polar solvent is improved due to charge repulsion between functional groups in the polar solvent, aggregation is reduced, and the fiber structure tends to be uniformly coated. On the other hand, when the degree of oxidation (O/C ratio) of the graphene oxide is 0.8 or less, the conductivity of the graphene obtained by reduction hardly is to be poor.

If graphite has not been oxidized to the inside of the graphene oxide, it is difficult to obtain a flaky graphene when the graphene oxide is reduced. Therefore, it is desirable that no peak specific to graphite is detected when graphene oxide is dried and subjected to X-ray diffraction measurement.

When a graphene dispersion liquid is used as a dispersion liquid for coating, the degree of oxidation (O/C ratio) of graphene to be used is preferably 0.05 or more, more preferably 0.07 or more, even more preferably 0.09 or more, and further preferably 0.10 or more. The degree of oxidation (O/C ratio) is preferably 0.35 or less, more preferably 0.30 or less, even more preferably 0.20 or less, and further preferably 0.15 or less. When the degree of oxidation (O/C ratio) is 0.05 or more, graphene can be charged in a solvent, and dispersibility in the solvent tends to be improved. On the other hand, when the degree of oxidation (O/C ratio) is 0.35 or less, the $\pi$-electron conjugated structure is restored by reduction of graphene, so that conductivity is obtained.

By dispersing such a graphene material in a solvent, a dispersion liquid of the graphene material is obtained. As the solvent, a polar solvent is preferable, and water, ethanol, methanol, 1-propanol, 2-propanol, N-methylpyrrolidone, dimethylformamide, dimethylacetamide, dimethylsulfoxide, $\gamma$-butyrolactone, mixtures thereof can be used. It is particularly preferable to use water. As an apparatus for dispersing the graphene material in the solvent, it is preferable to use an apparatus having a strong shear force, such as a bead mill, a homogenizer, FILMIX (registered trademark) (PRIMIX

Corporation), a wet jet mill, a dry jet mill, or ultrasonic waves.

The concentration of the graphene material dispersion liquid is preferably 2% by weight or less, more preferably 1% by weight or less, and even more preferably 0.5% by weight or less from the viewpoint of permeability into the fiber structure. In order to increase the collision probability between the fiber structure and the graphene material, the concentration of the graphene material dispersion liquid is preferably 0.001% by weight or more, more preferably 0.01% by weight or more, and even more preferably 0.1% by weight or more.

In Step (i), it is also preferable to treat the surface of the fiber structure so as to be paired with the charge of the graphene material before coating with the graphene material. Since the graphene material has a negative charge, if the surface of the fiber structure has been cationized, the fiber structure can be easily coated with graphene by electrostatic adsorption.

Examples of the method for cationizing the surface of the fiber structure include a method comprising bringing the surface of the fiber structure into contact with a solution containing a cationizing agent. Examples of the method of bringing the surface of the fiber structure into contact with the solution containing the cationizing agent include a method of immersing the fiber structure in the solution containing the cationizing agent.

Examples of the cationizing agent that can be used include a cationic low molecular weight compound such as a quaternary ammonium salt and a pyridinium salt, and a cationic polymer compound. It is preferable to use a cationic polymer compound because the coating of the conductive material can be made uniform.

In Step (ii), a silicone hydrogel precursor solution comprising a monomer represented by Formula (I) is brought into contact with the conductive substrate layer thus obtained. As the monomer contained in the precursor solution, the above-mentioned preferable monomer may be contained in the above-mentioned preferable composition. Examples of the method of bringing the conductive substrate layer into contact with the precursor solution of a silicone hydrogel include a method comprising immersing the conductive substrate layer in the precursor solution, and a method comprising placing the conductive substrate layer in a mold and pouring the precursor solution.

The precursor solution preferably comprises a polymerization initiator. Examples of a suitable initiator include thermal polymerization initiators such as peroxides and azo compounds, photopolymerization initiators (including UV light, visible light, or a combination), or mixtures thereof. When thermal polymerization is performed, a thermal polymerization initiator having optimum decomposition characteristics for a desired reaction temperature is selected and used. In general, an azo-based initiator or a peroxide-based initiator having a 10 hour half-life temperature of 40°C or higher and 120°C or lower is preferable. Examples of the photopolymerization initiator include carbonyl compounds, peroxides, azo compounds, sulfur compounds, halogen compounds, and metal salts.

More specific examples of the photopolymerization initiator include aromatic $\alpha$-hydroxyketone, alkoxyoxybenzoin, acetophenone, acylphosphine oxide, bisacylphosphine oxide, and mixtures thereof. More specific examples of the photopolymerization initiator include 1-hydroxycyclohexyl phenyl ketone, 2-hydroxy-2-methyl-1-phenyl-propan-1-one, bis(2,6-dimethoxybenzoyl)-2,4,4-trimethylpentylphosphine oxide (DMBAPO), bis(2,4,6-trimethylbenzoyl)-phenylphosphine oxide ("IRGACURE" (registered trademark) 819), 2,4,6-trimethylbenzyldiphenylphosphine oxide, 2,4,6-trimethylbenzoyldiphenylphosphine oxide, and benzoin methyl ether.

Examples of commercially available visible light polymerization initiator systems include "IRGACURE" (registered trademark) 819, "IRGACURE" (registered trademark) 1700, "IRGACURE" (registered trademark) 1800, "IRGACURE" (registered trademark) 1850 (manufactured by BASF), and Lucirin TPO initiator (manufactured by BASF). Examples of a commercially available UV photopolymerization initiator include "DAROCUR" (registered trademark) 1173 and "DAROCUR" (registered trademark) 2959 (manufactured by BASF). These polymerization initiators may be used singly or in combination. The amount of the polymerization initiator used is preferably in a range of 0.1% by weight or more and 1% by weight or less with respect to the total weight of all monomer components.

The precursor solution preferably comprises a polymerization solvent. As the polymerization solvent, various solvents such as an organic solvent and an inorganic solvent can be applied. Examples thereof include water, alcohol solvents such as methanol, ethanol, propanol, 2-propanol, butanol, tert-butanol, tert-amyl alcohol, and 3,7-dimethyl-3-octanol and tetrahydrolinalool; aromatic hydrocarbon-solvents such as benzene, toluene, and xylene; aliphatic hydrocarbon-solvents such as hexane, heptane, octane, decane, petroleum ether, kerosene, ligroin, and paraffin; ketone solvents such as acetone, methyl ethyl ketone, and methyl isobutyl ketone; ester solvents such as ethyl acetate, butyl acetate, methyl benzoate, dioctyl phthalate, and ethylene glycol diacetate; and glycol ether solvents such as diethyl ether, tetrahydrofuran, dioxane, ethylene glycol dialkyl ether, diethylene glycol dialkyl ether, triethylene glycol dialkyl ether, tetraethylene glycol dialkyl ether, polyethylene glycol dialkyl ether, polyethylene glycol-polypropylene glycol block copolymers, and polyethylene glycol-polypropylene glycol random copolymers. These solvents may be used singly or in combination. Among them, a solvent selected from among water, tert-butanol, tert-amyl alcohol, and 3,7-dimethyl-3-octanol is preferable in that radical polymerization is hardly inhibited.

Next, in Step (iii), the precursor solution is polymerized to form a silicone hydrogel layer, and thus a conductive sheet comprising a conductive substrate layer and a silicone hydrogel layer is obtained.

The polymerization of the precursor solution can be performed by a known technique. Specific examples thereof

include photopolymerization in which a precursor solution is irradiated with active light rays such as ultraviolet rays, visible light rays, or a combination thereof, and heating polymerization in which a precursor solution is placed in an oven or a liquid vessel and heated to polymerize. There may be a method of using both techniques in combination, such as heat polymerization is performed after photopolymerization, or photopolymerization is performed after heat polymerization. Commonly, in the case of photopolymerization, light from a light source such as a mercury lamp or a fluorescent lamp is radiated for a short period (usually for not more than 1 hour). Photopolymerization is preferably used because of its superior processability.

EXAMPLES

The present invention will be specifically described with reference to Examples.

Hereinafter, methods for measuring various physical properties of graphene will be described.

(Measurement of Average Size and Average Thickness of Graphene on Fiber Structure)

A fiber structure coated with graphene was immersed in water at a bath ratio of 1:50. Graphene was liberated from the fiber structure by stirring at 40°C and a rotation speed of 450 rpm for 24 hours using a hot stirrer REXIM RSH-6DN (AS ONE Corporation). The mixture obtained was subjected several times to suction filtration on filter paper having a pore diameter capable of passing through only the smaller one of the fiber and the graphene to separate the fiber structure and the graphene. Thereafter, the graphene obtained was diluted with N-methylpyrrolidone, and the diluted liquid was treated at a rotation speed of 40 m/s (shear rate: 20,000 $sec^{-1}$) for 60 seconds using FILMIX (registered trademark) model 30-30 (PRIMIX Corporation), and then the diluted liquid was dropped on a mica substrate, followed by drying, so that graphene was attached to the substrate. The graphene on the substrate was observed with a laser microscope VK-X250 manufactured by Keyence Corporation, and the length of the longest part (long diameter) and the length of the shortest part (short diameter) of randomly selected 50 small pieces of the graphene were measured, and the average of 50 values that are each obtained by (long diameter + short diameter)/2 was obtained.

In addition, the graphene on the substrate was observed with an atomic force microscope (Dimension Icon; manufactured by Bruker Corporation). The thickness of the graphene was measured for randomly selected 50 small pieces and the average value was calculated. When there was a variation in thickness in one piece, the area average of the thickness was determined.

(Measurement of Thickness of Graphene Coating Layer)

A cross section of a graphene-coated fiber structure was prepared using an ion milling (Arblade 5000; manufactured by Hitachi High-Technologies Corporation) and the cross section was observed with a field emission electron microscope (JEM-2100 F; manufactured by JEOL Ltd.), and thus the thickness of the graphene coating layer was measured. The thicknesses at 10 locations were measured and the average was determined.

(Measurement of Degree of Oxidation (O/C Ratio) of Graphene)

Graphene separated from a graphene-coated fiber structure produced in Example was analyzed by X-ray photoelectron spectroscopy, and the degree of oxidation (O/C ratio) of the graphene was measured.

Measurement was performed using Quantera SXM (manufactured by PHI, Inc.). The excited X-ray was monochromatic Al $K\alpha$ 1, 2 rays (1486.6 eV), the X-ray diameter was 200 $\mu$m, and the photoelectron take-off angle was 45°. The peak around 284.3 eV was assigned to a C1s peak based on carbon atoms, the peak around 533 eV was assigned to an O1s peak based on oxygen atoms, and the O/C ratio was determined from the area ratio of the peaks.

The method for separating graphene from the graphene-coated fiber structure was performed by the following procedure. The graphene-coated structure was immersed in water at a bath ratio of 1:50, and graphene was liberated by stirring at a temperature of 40°C and a rotation speed of 450 rpm for 24 hours using a hot stirrer REXIM RSH-6DN (AS ONE Corporation). The mixture obtained was subjected several times to suction filtration on filter paper having a pore diameter capable of passing through only the smaller one of the fiber and the graphene to separate the fiber structure and the graphene. Thereafter, the graphene obtained was diluted with N-methylpyrrolidone, and the diluted liquid was treated at a rotation speed of 40 m/s (shear rate: 20,000 $sec^{-1}$) for 60 seconds using FILMIX (registered trademark) model 30-30 (PRIMIX Corporation), and then the diluted liquid was filtered with a suction filter, followed by freeze drying, so that graphene was obtained.

(Measurement of thickness of conductive substrate layer)

The thickness of the conductive substrate layer was determined from the average of five measured values using a digimatic micrometer (manufactured by Mitutoyo Corporation).
The methods for measuring physical properties are as follows.

(Measurement of moisture content of silicone hydrogel)

Silicone hydrogel was extracted from the conductive sheet. The silicone hydrogel extracted was immersed in distilled water at 25°C for 24 hours, and the test piece was taken out from the distilled water, then the distilled water on the surface was lightly wiped with a clean cloth within 1 minute, and immediately after that, the weight (W1) of the silicone hydrogel in a hydrous state was weighed. Subsequently, the silicone hydrogel in a hydrous state was dried in a vacuum dryer at 40°C for 16 hours, and then the silicone hydrogel was taken out from the vacuum dryer, and the weight (W2) in a dry state was weighed within 1 minute, and the water content was calculated by the following formula.

$$\text{Water content (\%)} = (W1 - W2) / W1 \times 100$$

(Evaluation of Adhesiveness)

The surface tack strength of a conductive sheet in a dry state was measured using a rheometer CR-500DX manufactured by Sun Scientific Co., Ltd. A conductive sheet having a diameter of 40 mm and a thickness of 1 mm obtained in Example was brought into a dry state, and then fixed to the stage of the rheometer within 1 minute, and a probe having a stainless steel disk (diameter: 10 mm) at the tip was brought into contact with the hydrogel layer of the conductive sheet at a load of 50 N, and then the stage was moved at a speed of 50 mm/min. A load applied when the probe was peeled off from the hydrogel layer was measured. The probe was brought into contact with a clean surface of the silicone hydrogel. The measurement was continuously performed three times, and the average value thereof was calculated and taken as the surface tack strength.

(Measurement of Air Permeability of Conductive Substrate Layer)

In the same manner as in each Example, a conductive substrate layer having a size of 100 mm × 100 mm was prepared, and the airflow resistance R (kPa·s/m) was measured using KES-F8-AP1 manufactured by Kato Tech Co., Ltd. From the results obtained, the air permeability Q ($cm^3/cm^2/s$) was calculated using Q = 12.5/R based on JIS L 1096 (2010) Air permeability A method.

(Measurement of Minimum Bending Radius of Conductive Substrate Layer)

A conductive substrate layer obtained in each Example was wound such that it came into close contact with a piano wire (EA951AP-105 manufactured by ESCO Co., Ltd.) having a thickness of 0.5 mm in diameter, and whether or not irreversible breakage such as a crack or a fold occurred in the substrate at that time was observed. At this time, when no breakage was observed, it was judged that the minimum bending radius was smaller than 0.25 mm, and the result was described as "< 0.25 mm".

(Measurement of impedance of conductive substrate layer)

A conductive substrate layer prepared in the same manner as in each Example was cut into a rectangular shape having a width of 10 mm and a length of 110 mm, and a silver paste (DOTITE (registered trademark) D-500 manufactured by Fujikura Kasei Co., Ltd.) was applied to both ends 5 mm in the longitudinal direction and dried. Alligator clips were attached to the ends to which the silver paste had been applied, and the impedance was measured using MultiStat (Model 1480A manufactured by Solartron) and a frequency analyzer (Model 1255B manufactured by Solartron) with the conductive substrate layer flattened. The measurement conditions were Amplitude = 10 mV and Frequency = 0.1 Hz to 1 kHz. In the conductive sheet of the present invention, since the frequency dependence of impedance was not observed, the value of the impedance at 1 Hz was entered in Table 1 as a representative value.

(Evaluation of electrocardiographic measurement of conductive sheet)

An AD8232 Heart Rate Monitor (manufactured by SparkFun Electronics) and a Sensor Cable-Electro Pads

(manufactured by SparkFun Electronics) were connected to an Arduino Pro Mini 328 (manufactured by SparkFun Electronics), and an FTDI Basic Breakout (manufactured by SparkFun Electronics) was connected to a computer as a USB interface. The conductive sheet of the present invention was connected to each of the three tips of the Sensor Cable-Electro Pads and stuck to three positions in total, namely, the left and right upper arms and the flank, and whether electrocardiography could be performed was judged. When the electrocardiogram was measured, it was determined as "Possible", and when the electrocardiogram was not measured, it was determined as "Impossible".

(Evaluation of biocompatibility)

Five males in their twenties to fifties were put on a conductive sheet obtained in Example for 6 hours, and reported the presence or absence of discomfort. When half or more of the persons answered that there was no discomfort, it was determined that the discomfort was "absent", and when less than half answered so, it was determined that the discomfort was "present". In the present invention, the biocompatibility is evaluated to be high when there is no discomfort in such an evaluation, and the biocompatibility is evaluated to be low when there is discomfort.

(Measurement of Tensile Elastic Modulus of Silicone Hydrogel in Dry State)

The hydrogel was extracted from the conductive sheet in a hydrous state by excision, and an array type sample having a width of 5 mm at the narrowest part was prepared. The sample was dried in a vacuum dryer at 40°C for 16 hours or more to be in a dry state. The width of the narrowest part of the sample in a dry state was measured with a caliper. The thickness was measured using an ABC Digimatic Indicator (ID-C 112, manufactured by Mitutoyo Corporation), and the cross-sectional area was calculated. Next, a tensile test of the sample was performed using a Tensilon (RTM-100 manufactured by Toyo Baldwin Co., Ltd., crosshead speed: 100 mm/min), and the tensile elastic modulus was calculated using the cross-sectional area of the narrowest part of the sample.

[Abbreviation]

The abbreviations of the monomers, etc. used in Examples are shown below.

MEA: 2-methoxyethyl acrylate
mPDMS: monofunctional linear silicone monomer (average molecular weight: 1000, MCR-M11 manufactured by Gelest, Inc.)
DMA: N,N-dimethylacrylamide manufactured by Tokyo Chemical Industry Co., Ltd.
164A: bifunctional silicone monomer, X-22-164A manufactured by Shin-Etsu Chemical Co., Ltd., functional group equivalent: 860
IC: photopolymerization initiator "IRGACURE" (registered trademark) 819 manufactured by Ciba Specialty Chemicals
TAA: tert-amyl alcohol manufactured by Tokyo Chemical Industry Co., Ltd.

[Example 1]

(Preparation of Aqueous Graphene Oxide Dispersion)

A 1500 mesh natural graphite powder (Shanghai Yifan Graphite Co., Ltd.) was used as a raw material. To 10 g of natural graphite powder in an ice bath were added 220 ml of 98% concentrated sulfuric acid, 5 g of sodium nitrate, and 30 g of potassium permanganate, and the mixture was mechanically stirred for 1 hour. The temperature of the mixed solution was kept at 20°C or lower. The mixed solution was taken out from the ice bath, and reacted by stirring in a water bath at 35°C for 4 hours. Thereafter, the suspension obtained by adding 500 ml of ion-exchanged water was further reacted at 90°C for 15 minutes. Thereafter, 600 ml of ion-exchanged water and 50 ml of hydrogen peroxide were added, and a reaction was performed for 5 minutes to obtain a graphene oxide dispersion liquid. This was filtered while it was hot, and the graphene oxide obtained was washed with a dilute hydrochloric acid solution to remove metal ions. Further, washing was repeated with ion-exchanged water until the pH reached 7 to remove the acid, thereby preparing a graphene oxide gel. The element ratio of oxygen atoms to carbon atoms (O/C ratio) of the prepared graphene oxide gel measured by X-ray photoelectron spectroscopy was 0.53. Ion exchanged water was added to the obtained graphene oxide gel to a concentration of 0.5% by weight, and ultrasonic waves were applied at an output of 300 W for 5 minutes using an ultrasonic apparatus UP400S (Hielscher Ultrasonics GmbH), and thus a 0.5% by weight aqueous graphene oxide dispersion was prepared. The graphene oxide obtained had an average size of 3 $\mu$m in a direction parallel to the graphene layer, an average thickness of 3 nm, and an O/C ratio of 0.7.

(Cationization Treatment of PET Nonwoven Fabric)

A PET nonwoven fabric made of PET fibers having a yarn diameter of 12.4 μm was cut into a circle having a diameter of 40 mm with a protruding rectangular portion having a width of 10 mm and a length of 15 mm. In order to perform a cationization treatment, the PET nonwoven fabric was immersed in a 1% by weight aqueous polyethyleneimine (number average molecular weight: 70,000) solution at a bath ratio of 1:50, and treated at a temperature of 60°C and a rotation speed of 450 rpm for 30 minutes using a hot stirrer REXIM RSH-6DN (AS ONE Corporation). Thereafter, an operation of washing the cationized PET nonwoven fabric in ion-exchanged water at a bath ratio of 1:1000, room temperature, and a rotation speed of 200 rpm for 1 minute using a stirrer was repeated twice.

Next, in order to coat with graphene, the PET nonwoven fabric cationized as described above was immersed in the 0.5% by weight aqueous graphene oxide dispersion liquid prepared as described above at a bath ratio of 1:50, and treated at a temperature of 60°C and a rotation speed of 450 rpm for 30 minutes using a hot stirrer. Thereafter, an operation of washing the cationized PET nonwoven fabric in ion-exchanged water at a bath ratio of 1:1000, room temperature, and a rotation speed of 600 rpm for 1 minute using a stirrer was repeated three times.

Subsequently, the circular PET nonwoven fabric obtained as described above was immersed in a 5% by weight aqueous sodium dithionite solution at a bath ratio of 1:50, and stirred at a temperature of 40°C and a rotation speed of 450 rpm for 5 minutes using a hot stirrer to perform a reduction treatment. Thereafter, an operation of washing the cationized PET nonwoven fabric in ion-exchanged water at a bath ratio of 1:1000, room temperature, and a rotation speed of 600 rpm for 1 minute using a stirrer was repeated three times. Thereafter, the circular PET nonwoven fabric was dried in a hot air dryer at 60°C to obtain a circular graphene-coated PET nonwoven fabric. For the graphene-coated PET nonwoven fabric obtained, the thickness of the graphene coating layer, the average size and the average thickness of graphene, the O/C ratio, the thickness, the air permeability, the minimum bending radius, and the impedance of graphene were measured and shown in Table 1.

A monomer composition was obtained by thoroughly mixing 100 mg (10% by weight) of MEA, 500 mg (50% by weight) of mPDMS, 300 mg (30% by weight) of DMA, 100 mg (10% by weight) of 164A, 2 mg (0.2% by weight) of IC, and 400 mg (40% by weight) of TAA. The % by weight of each component is a value calculated where the sum total of the monomer components forming the silicone hydrogel excluding the solvent and the polymerization initiator was 100% by weight. This monomer composition was injected into a circular mold made of a silicone resin and having a diameter of 40 mm to have a liquid height of 1 mm, and the circular graphene-coated PET nonwoven fabric obtained as described above was immersed in the monomer composition. Light irradiation (1.01 $mW/cm^2$, 30 min) was applied using a fluorescent lamp (TOSHIBA CORPORATION, FL-6D, daylight color, 6 W, 4 lamps), thereby polymerizing the monomer composition. After the polymerization, the silicone hydrogel molded article was immersed in a mixed solution of isopropyl alcohol:water = 1:1 together with the mold, allowed to stand at room temperature for 1 hour, and then peeled off from the mold. The molded article obtained was immersed in a new mixed solution of isopropyl alcohol:water = 1:1 at 60°C for 1 hour, then immersed in distilled water, and allowed to stand overnight and preserved. The molded article was taken out from the distilled water and thoroughly washed with water to afford a conductive sheet in which the graphene-coated PET nonwoven fabric was embedded in the silicone hydrogel. A silver paste was applied to the protruding rectangular portion of the molded article and dried, and then an alligator clip was attached, and the molded body was connected to an electrocardiograph to perform electrocardiographic measurement. The results are shown in Table 2.

[Examples 2 to 7]

Monomer compositions were prepared according to the composition ratios entered in Table 2, and conductive sheets were prepared in the same manner as in Experimental Example 1. All of the samples obtained had good surface tackiness, and electrocardiography could be performed without discomfort feeling.

[Example 8]

An aluminum foil (50 μm thick, manufactured by Hohsen Corporation) was cut into a circle having a diameter of 40 mm with a protruding rectangular portion having a width of 10 mm and a length of 15 mm, and the circular portion was subjected to punching with a diameter of 6 mm at 6 locations. The same procedures as in Example 7 were carried out except that the graphene-coated PET nonwoven fabric of Example 7 was changed to the aluminum foil subjected to the punching.

[Example 9]

A PET film was used instead of the aluminum foil, and the PET film was cut and punched in the same manner as in Example 8. This film was immersed in a 0.5% by weight aqueous graphene oxide dispersion liquid, then dried,

and then immersed in a 1% by weight aqueous hydrazine solution for 30 minutes to perform reduction. After washing with ion-exchanged water, it was dried to prepare a conductive substrate layer having air permeability. This was embedded in a silicone hydrogel in the same manner as in Example 7, subjected to the same extraction and washing treatment, and then subjected to electrocardiography.

[Example 10]

The same procedures were carried out as in Example 7, except that the PET nonwoven fabric of Example 7 was replaced by a PET woven fabric (woven fabric composed of 84T-36F polyester filaments having a single fiber diameter of 15 μm and a multifilament diameter of 243 μm and having a warp pitch of 520 μm and a weft pitch of 520 μm).

[Example 11]

The same procedures were carried out except that the PET nonwoven fabric of Example 7 was replaced with a PET knitted fabric (yarn diameter: 15 μm, perimeter: 47 μm).

[Example 12]

A PET nonwoven fabric was cut into the same shape as that of Example 1, immersed in a 1.3% by weight aqueous PEDOT:PSS solution (manufactured by Sigma-Aldrich Co. LLC), and then dried to prepare a conductive substrate layer having air permeability. This was embedded in a silicone hydrogel in the same manner as in Example 7, subjected to the same extraction and washing treatment, and then subjected to electrocardiography.

[Example 13]

The same procedures were carried out as in Example 12, except that the PEDOT:PSS of Example 12 was replaced by carbon nanotubes (manufactured by Sigma-Aldrich Co. LLC).

[Example 14]

The same procedures were carried out as in Example 12, except that the PEDOT:PSS of Example 12 was replaced by DOTITE (registered trademark) D-500 manufactured by Fujikura Kasei Co., Ltd. diluted 100 times with toluene.

[Example 15]

The same procedures were carried out in the same manner except that the aluminum foil of Example 8 was not subjected to punching. The resulting conductive sheet could be used for electrocardiography at a low water content of 30% and had good surface tack strength, but exhibited stuffy feeling and discomfort due to lack of air permeability.

[Comparative Example 1]

The same procedures were carried out in the same manner except that the monomer composition of the silicone hydrogel of Example 1 was changed to 10% by weight of acrylic acid, 18% by weight of DMA, 2% by weight of methylenebisacrylamide, 40% by weight of TAA, and 0.2% by weight of IC. Since the conductive sheet did not have a layer formed of a silicone hydrogel, adhesiveness was insufficient, so that the conductive sheet easily fell off from the skin and it is difficult to perform electrocardiography.

[Comparative Example 2]

The same procedures were carried out in the same manner except that the monomer composition of the silicone hydrogel of Example 1 was changed to 60% by weight of mPDMS, 30% by weight of DMA, 10% by weight of 164A, 40% by weight of TAA, and 0.2% by weight of IC. Since the hydrogel layer of the conductive sheet did not contain the repeat unit (A) derived from a monomer represented by Formula (I), adhesiveness was insufficient and the hydrogel layer easily fell off from the skin, so that electrocardiography was difficult to measure.

[Table 1-1]

| | Conductive substrate layer | Conductive material | Thickness of graphene coating layer (nm) | Size of graphene in direction parallel to graphene layer (μm) |
|---|---|---|---|---|
| Example 1 | PET nonwoven fabric | Graphene | 13 | 3 |
| Example 2 | PET nonwoven fabric | Graphene | 13 | 3 |
| Example 3 | PET nonwoven fabric | Graphene | 13 | 3 |
| Example 4 | PET nonwoven fabric | Graphene | 13 | 3 |
| Example 5 | PET nonwoven fabric | Graphene | 13 | 3 |
| Example 6 | PET nonwoven fabric | Graphene | 13 | 3 |
| Example 7 | PET nonwoven fabric | Graphene | 13 | 3 |
| Example 8 | Aluminum foil | None | - | - |
| Example 9 | PET film | Graphene | 21 | 3 |
| Example 10 | PET woven fabric | Graphene | 8 | 3 |
| Example 11 | PET knitted fabric | Graphene | 10 | 3 |
| Example 12 | PET nonwoven fabric | PEDOT:PSS | - | - |
| Example 13 | PET nonwoven fabric | CNT | - | - |
| Example 14 | PET nonwoven fabric | Silver particles | - | - |
| Example 15 | Aluminum foil (No punching) | None | - | - |
| Comparative Example 1 | PET nonwoven fabric | Graphene | 13 | 3 |
| Comparative Example 2 | PET nonwoven fabric | Graphene | 13 | 3 |

[Table 1-2]

| | O/C ratio | Thickness of conductive substrate Layer (mm) | Air permeability Q ($cm^3/cm^2 \cdot s$) | Minimum bending radius (mm) | Impedance (Ω) |
|---|---|---|---|---|---|
| Example 1 | 0.10 | 0.14 | 300 | < 0.25 | $3.0 \times 10^4$ |
| Example 2 | 0.10 | 0.14 | 300 | < 0.25 | $3.0 \times 10^4$ |
| Example 3 | 0.10 | 0.14 | 300 | < 0.25 | $3.0 \times 10^4$ |
| Example 4 | 0.10 | 0.14 | 300 | < 0.25 | $3.0 \times 10^4$ |

(continued)

| | O/C ratio | Thickness of conductive substrate Layer (mm) | Air permeability Q ($cm^3/cm^2 \cdot s$) | Minimum bending radius (mm) | Impedance ($\Omega$) |
|---|---|---|---|---|---|
| Example 5 | 0.10 | 0.14 | 300 | < 0.25 | $3.0 \times 10^4$ |
| Example 6 | 0.10 | 0.14 | 300 | < 0.25 | $3.0 \times 10^4$ |
| Example 7 | 0.10 | 0.14 | 300 | < 0.25 | $3.0 \times 10^4$ |
| Example 8 | - | 0. 05 | 431 | < 0.25 | $5.6 \times 10^{-2}$ |
| Example 9 | 0.09 | 0.15 | 418 | < 0.25 | $6.4 \times 10^3$ |
| Example 10 | 0.11 | 0.09 | 128 | < 0.25 | $2.4 \times 10^4$ |
| Example 11 | 0.13 | 0.38 | 87 | < 0.25 | $1.6 \times 10^4$ |
| Example 12 | - | 0.14 | 300 | < 0.25 | $3.5 \times 10^6$ |
| Example 13 | - | 0.14 | 300 | < 0.25 | $4.6 \times 10^{-1}$ |
| Example 14 | - | 0.14 | 300 | < 0.25 | $1.7 \times 10^1$ |
| Example 15 | - | 0.05 | 0 | < 0.25 | $5.6 \times 10^{-2}$ |
| Comparative Example 1 | 0.10 | 0.14 | 300 | < 0.25 | $3.0 \times 10^4$ |
| Comparative Example 2 | 0.10 | 0.14 | 300 | < 0.25 | $3.0 \times 10^4$ |

[Table 2-1]

| | MEA (Repeat unit (A)) | mPDMS(Repeat unit (B)) | DMA (Repeat unit (C)) | X-22-164A (Crosslinking component) | Silicon atom content |
|---|---|---|---|---|---|
| | (wt%) | (wt%) | (wt%) | (wt%) | (wt%) |
| Example 1 | 10 | 50 | 30 | 10 | 18.9 |
| Example 2 | 10 | 70 | 10 | 10 | 25.1 |
| Example 3 | 30 | 30 | 30 | 10 | 12.7 |
| Example 4 | 30 | 50 | 10 | 10 | 18.9 |
| Example 5 | 50 | 10 | 30 | 10 | 6.6 |
| Example 6 | 70 | 10 | 10 | 10 | 6.6 |
| Example 7 | 20 | 45 | 25 | 10 | 17.4 |
| Example 8 | 20 | 45 | 25 | 10 | 17.4 |
| Example 9 | 20 | 45 | 25 | 10 | 17.4 |
| Example 10 | 20 | 45 | 25 | 10 | 17.4 |
| Example 11 | 20 | 45 | 25 | 10 | 17.4 |
| Example 12 | 20 | 45 | 25 | 10 | 17.4 |
| Example 13 | 20 | 45 | 25 | 10 | 17.4 |
| Example 14 | 20 | 45 | 25 | 10 | 17.4 |
| Example 15 | 20 | 45 | 25 | 10 | 17.4 |
| Comparative Example 1 | 10% by weight of acrylic acid, 18% by weight of DMA, and 2% by weight of methylenebisacrylamide | | | | 0.0 |

(continued)

| | MEA (Repeat unit (A)) | mPDMS(Repeat unit (B)) | DMA (Repeat unit (C)) | X-22-164A (Crosslinking component) | Silicon atom content |
|---|---|---|---|---|---|
| | (wt%) | (wt%) | (wt%) | (wt%) | (wt%) |
| Comparative Example 2 | 0 | 60 | 30 | 10 | 22.0 |

[Table 2-2]

| | Moisture content | Surface tack strength | Discomfort | Electrocardiography | Tensile elastic modulus in dry state |
|---|---|---|---|---|---|
| | (wt%) | (N) | Present/ Absent | Possible/ Impossible | (MPa) |
| Example 1 | 60 | 7.7 | Absent | Possible | 2.2 |
| Example 2 | 35 | 3.4 | Absent | Possible | 1.6 |
| Example 3 | 55 | 15.2 | Absent | Possible | 0.9 |
| Example 4 | 48 | 6.8 | Absent | Possible | 0.8 |
| Example 5 | 34 | 14.9 | Absent | Possible | 0.7 |
| Example 6 | 18 | 1.6 | Absent | Possible | 0.5 |
| Example 7 | 30 | 13.1 | Absent | Possible | 1.3 |
| Example 8 | 30 | 13.1 | Absent | Possible | 1.3 |
| Example 9 | 30 | 13.1 | Absent | Possible | 1.3 |
| Example 10 | 30 | 13.1 | Absent | Possible | 1.3 |
| Example 11 | 30 | 13.1 | Absent | Possible | 1.3 |
| Example 12 | 30 | 13.1 | Absent | Possible | 1.3 |
| Example 13 | 30 | 13.1 | Absent | Possible | 1.3 |
| Example 14 | 30 | 13.1 | Absent | Possible | 1.3 |
| Example 15 | 30 | 13.1 | Present | Possible | 1.3 |
| Comparative Example 1 | 67 | 0 | Absent | Impossible | 12.2 |
| Comparative Example 2 | 56 | 0 | Absent | Impossible | 9.6 |

## Claims

1. A conductive sheet comprising a conductive substrate layer and a hydrogel layer formed of a silicone hydrogel having a polymer comprising a repeat unit (A) derived from a monomer represented by Formula (I) as a gel skeleton:

[Chemical Formula 1]

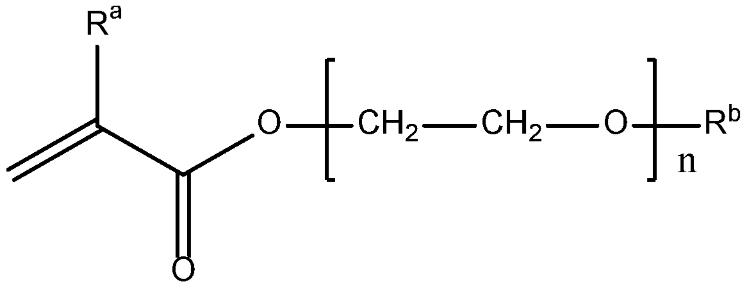

wherein, in Formula (I), $R^a$ represents hydrogen or a methyl group, $R^b$ represents an organic group having from 1 to 15 carbon atoms, and n represents an integer within a range of 1 to 40.

**2.** The conductive sheet according to claim 1, wherein the silicone hydrogel comprises the repeat unit (A) derived from the monomer represented by Formula (I) in an amount within a range of 10% by weight to 70% by weight with respect to the total weight of the silicone hydrogel in a dry state.

**3.** The conductive sheet according to claim 1 or 2, wherein the polymer comprising the repeat unit (A) further comprises a repeat unit (B) derived from a monofunctional silicone monomer.

**4.** The conductive sheet according to claim 3, wherein the silicone hydrogel comprises the repeat unit (B) in an amount within a range of 10% by weight to 70% by weight with respect to the total weight of the silicone hydrogel in a dry state.

**5.** The conductive sheet according to any one of claims 1 to 4, wherein the polymer comprising the repeat unit (A) further comprises a repeat unit (C) derived from a monomer containing an amide structure.

**6.** The conductive sheet according to claim 5, wherein the silicone hydrogel comprises the repeat unit (C) in an amount within a range of 10% by weight to 50% by weight with respect to the total weight of the silicone hydrogel in a dry state.

**7.** A conductive sheet comprising a conductive substrate layer and a hydrogel layer comprising a silicone hydrogel, wherein the hydrogel layer has a tensile elastic modulus within a range of 0.1 MPa to 3.5 MPa in a dry state.

**8.** The conductive sheet according to any one of claims 1 to 7, wherein the conductive substrate layer has an impedance of $10^{-3}$ Ω to $10^{7}$ Ω at 0.1 Hz to 1000 Hz.

**9.** The conductive sheet according to any one of claims 1 to 8, wherein the hydrogel layer has a surface tack strength of 1 N to 30 N.

**10.** The conductive sheet according to any one of claims 1 to 9, wherein the conductive substrate layer is embedded in the hydrogel layer.

**11.** The conductive sheet according to any one of claims 1 to 10, wherein the conductive substrate layer has air permeability.

**12.** The conductive sheet according to any one of claims 1 to 11, wherein the conductive substrate layer comprises a fiber structure coated with a conductive material, the fiber structure is selected from the group consisting of woven fabric, knitted fabric, lace, and nonwoven fabric.

**13.** The conductive sheet according to any one of claims 1 to 12, wherein the conductive substrate layer is a fiber structure comprising a fiber comprising graphene.

**14.** The conductive sheet according to claim 13, wherein the conductive substrate layer is a fiber structure comprising a fiber coated with graphene.

**15.** The conductive sheet according to any one of claims 13 to 14, wherein the graphene coating layer in the fiber coated

with the graphene has a thickness of 1 nm to 100 nm.

**16.** The conductive sheet according to any one of claims 13 to 15, wherein the graphene has a size of 0.10 μm to 10.00 μm in a direction parallel to the graphene layer.

**17.** The conductive sheet according to any one of claims 13 to 16, wherein the graphene has an element ratio of oxygen atoms to carbon atoms (O/C ratio) of from 0.05 to 0.35 as measured by X-ray photoelectron spectroscopy.

**18.** The conductive sheet according to any one of claims 1 to 17, wherein the conductive substrate layer has a thickness of 1 μm to 10 mm.

**19.** A method for producing a conductive sheet comprising a conductive substrate layer and a hydrogel layer formed of a silicone hydrogel having a polymer comprising a repeat unit (A) derived from a monomer represented by Formula (I) as a gel skeleton, the method comprising:

Step (ii): a step of bringing a silicone hydrogel precursor solution comprising a monomer represented by Formula (I) into contact with the conductive substrate layer; and
Step (iii): a step of polymerizing the silicone hydrogel.

**20.** The production method according to claim 19, wherein the method has, before Step (ii),
Step (i): a step of coating the fiber structure with graphene to obtain a conductive substrate layer.

**21.** The production method according to claim 20, wherein in Step (i), the fiber structure is coated with a dispersion of graphene oxide and then reduction treatment is performed to obtain the conductive substrate layer.

## INTERNATIONAL SEARCH REPORT

International application No. PCT/JP2020/010726

A. CLASSIFICATION OF SUBJECT MATTER
Int. Cl. B32B27/30(2006.01)i, B32B27/00(2006.01)i, D06M11/74(2006.01)i, D06M15/263(2006.01)i, D06M15/643(2006.01)i
FI: B32B27/30 A, B32B27/00 101, D06M11/74, D06M15/263, D06M15/643
According to International Patent Classification (IPC) or to both national classification and IPC

B. FIELDS SEARCHED
Minimum documentation searched (classification system followed by classification symbols)
Int. Cl. B32B27/30, B32B27/00, D06M11/74, D06M15/263, D06M15/643

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2018-50990 A (TORAY INDUSTRIES, INC.) 05 April 2018, claims 1, 2, 7, 8, paragraphs [0071], [0077], claims 1, 2, 7, 8, paragraphs [0020], [0022], [0071], [0077] | 1-8, 10, 19<br>11-18, 20-21 |
| Y | JP 2018-188788 A (TORAY INDUSTRIES, INC.) 29 November 2018, paragraph [0002], claims | 11-18, 20-21 |
| A | WO 2018/207586 A1 (TORAY INDUSTRIES, INC.) 15 November 2018 | 1-21 |
| A | WO 2017/170929 A1 (TOHOKU UNIVERSITY) 05 October 2017 | 1-21 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 20.05.2020 | 02.06.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2020/010726

C (Continuation). DOCUMENTS CONSIDERED TO BE RELEVANT

| Category* | Citation of document, with indication, where appropriate, of the relevant passages | Relevant to claim No. |
|---|---|---|
| A | JP 2002-542962 A (FIRST WATER LTD.) 17 December 2002 | 1-21 |
| A | JP 10-273881 A (TEIJIN LTD.) 13 October 1998 | 1-21 |

Form PCT/ISA/210 (continuation of second sheet) (January 2015)

**INTERNATIONAL SEARCH REPORT**
Information on patent family members

International application No.
PCT/JP2020/010726

| Patent Documents referred to in the Report | Publication Date | Patent Family | Publication Date |
|---|---|---|---|
| JP 2018-50990 A | 05.04.2018 | (Family: none) | |
| JP 2018-188788 A | 29.11.2018 | (Family: none) | |
| WO 2018/207586 A1 | 15.11.2018 | CN 110603061 A<br>TW 201900225 A | |
| WO 2017/170929 A1 | 05.10.2017 | (Family: none) | |
| JP 2002-542962 A | 17.12.2002 | US 2003/0054024 A1<br>GB 9909349 A<br>WO 2000/065143 A1<br>EP 1175527 A1<br>AU 4417300 A<br>CA 2367657 A<br>AT 247188 T<br>AU 765572 B | |
| JP 10-273881 A | 13.10.1998 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014157550 A
- JP 2015221947 A